# EUROPÄISCHE PATENTSCHRIFT

(11) **EP 2 403 364 B1**
(45) Veröffentlichungstag und Bekanntmachung des Hinweises auf die Patenterteilung: **09.01.2013**
(21) Anmeldenummer: 10708180.4
(22) Anmeldetag: 04.03.2010
(51) Int. Cl.: A23L 1/275, A23K 1/16, C07C 403/24

(54) **VERFAHREN ZUR HERSTELLUNG VON LÖSUNGEN VON ASTAXANTHIN-DERIVATEN**
METHOD FOR PRODUCING SOLUTIONS OF ASTAXANTHIN DERIVATIVES
PROCÉDÉ DE PRODUCTION DE SOLUTIONS DE DÉRIVÉS D'ASTAXANTHINE

(30) Priorität: 05.03.2009 EP 09154436
(43) Veröffentlichungstag der Anmeldung: 11.01.2012
(73) Patentinhaber: BASF SE, 67056 Ludwigshafen (DE)
(72) Erfinder: FELDTHUSEN JENSEN, Jesper, 55268 Nieder-Olm (DE); KÖPSEL, Christian, 69469 Weinheim (DE); BRANDS, Mario, 67063 Ludwigshafen (DE); ENGEL, Robert, 67346 Speyer (DE); PELLETIER, Wolf, 76879 Ottersheim (DE); END, Lutz, 68526 Ladenburg (DE); GOTTSCHALK, Thomas, 68165 Mannheim (DE)
(86) Internationale Anmeldenummer: PCT/EP2010/052763
(87) Internationale Veröffentlichungsnummer: WO 2010/100233

(56) Entgegenhaltungen:
- WO-A1-03/066583
- WO-A2-03/102116
- JP-A- 1 144 952

## Beschreibung

Die vorliegende Erfindung betrifft ein Verfahren zur Herstellung von Lösungen von Astaxanthin-Derivaten in für Nahrungsmittel geeigneten Ölen sowie ein Verfahren zur Herstellung von Futtermitteln.

Astaxanthin (3,3'-Dihydroxy-β,β-carotin-4,4'-dion) und seine Derivate, insbesondere seine Ester, sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördern deren Fruchtbarkeit. Zudem bewirken Astaxanthin und seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar.

Für verschiedene Anwendungszwecke, insbesondere zur Futtermittelherstellung, hat es sich grundsätzlich als vorteilhaft erwiesen, Astaxanthin oder seine Derivate in Form von verdünnten Lösungen in für Nahrungsmittel geeigneten, flüssigen Ölen bereitzustellen. Diese verdünnten Lösungen können dann bei der Futtermittelherstellung verwendet werden und gewährleisten eine hinreichende Bioverfügbarkeit in dem Futtermittel.

Bei der Herstellung derartiger Lösungen erweist sich die schlechte Lösungskinetik des Astaxanthins als problematisch.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst. Dieses Verfahren ist vergleichsweise aufwändig und bedarf größerer Mengen an Schutzkolloiden.

Die WO 2006/125591 beschreibt die Herstellung von öligen Carotinoid-Lösungen, beispielsweise Lösungen des Astaxanthins, wobei man zunächst eine Suspension des Carotinoids in der Ölphase bereitstellt, die Suspension für eine kurze Zeit auf hohe Temperaturen, z. B. im Bereich von 100 bis 230 °C erhitzt, um das Carotinoid in Lösung zu bringen, und die heiße Lösung mit einem kalten Öl vermischt. Zur Herstellung von öligen Lösungen des Astaxanthins werden regelmäßig Temperaturen oberhalb 160 °C angewendet. Dieses Verfahren ist mit einer Reihe von Nachteilen verbunden. Zum einen führt das Erhitzen aufgrund der chemischen Labilität von Carotinoiden unter anderem zu einer unerwünschten cis-trans-Isomerisierung der exocyclischen Doppelbindungen und damit zu einem Aktivitätsverlust. Zudem erweist sich die Bereitstellung öliger Suspensionen des Astaxanthins durch Vermahlen von Astaxanthin in Öl als problematisch.

Der vorliegenden Erfindung liegt die Aufgabe zu Grunde, ein Verfahren bereitzustellen, das es erlaubt, Astaxanthin oder das Astaxanthin-Derivat in ein für Nahrungsmittel geeignetes Öl einzuarbeiten, welches die Nachteile des Standes der Technik vermeidet, insbesondere ein Verfahren, das auch Anwendung erhöhter Temperaturen oder größerer Mengen an lipophilen Emulgatoren eine Einarbeitung des Astaxanthin-Derivats in ein für Nahrungsmittel geeignetes Öl ermöglicht.

Die ältere internationale Patentanmeldung PCT/EP2008/061384 beschreibt ein Verfahren zur Herstellung von öligen Lösungen des Astaxanthins in für Nahrungsmittel geeigneten Ölen, bei denen man bestimmte Astaxanthin-Derivate der im Folgenden angegebenen Formel I zunächst in eine Suspension in einem essbaren Öl überführt, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen. Diese Suspensionen sind typischerweise bei Temperaturen von 25 °C flüssig und können in einfacher Weise mit flüssigen, für Nahrungsmittel geeigneten Ölen verdünnt werden, wobei sich die verwendeten Astaxanthin-Derivate aufgrund ihrer vorteilhaften Lösungskinetik und der geringen Partikelgröße rasch in dem zur Verdünnung eingesetzten Öl auflösen, ohne dass es der Anwendung von Temperaturen oberhalb 100 °C und/oder größerer Mengen an Emulgatoren bedarf.

Es wurde überraschenderweise gefunden, dass die im Folgenden definierten Astaxanthin-Derivate der Formel I, sowie Astaxanthin-Derivate, welche zu wenigstens 70 Gew.-% aus wenigstens einer Verbindung der Formel I bestehen, die aus PCT/EP2008/061384 bekannten Vorteile auch dann aufweisen, wenn man sie in Form einer Lösung oder Suspension des Astaxanthin-Derivats in einem für Nahrungsmittel geeigneten organischen, aus Sauerstoff, Wasserstoff und Kohlenstoff bestehenden Lösungsmittel, das unter C₂-C₆-Alkanolen, C₁-C₄-Alkylestern aliphatischer C₁-C₄-Carbonsäuren und aliphatischen Ketonen mit 3 bis 6 C-Atomen ausgewählt ist, bereitstellt.

Die erfindungsgemäß eingesetzten Astaxanthin-Derivate bestehen zu wenigstens 70 Gew.-% aus einer Verbindung der Formel I 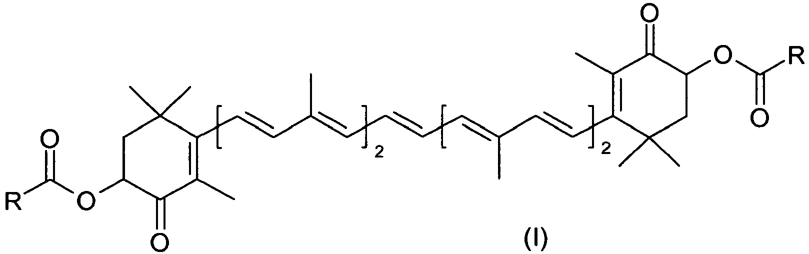 worin R für einen Rest der Formel 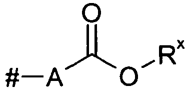 steht, worin # die Anknüpfung an die Carbonylgruppe bedeutet, A für -CH₂CH₂- oder für -CH=CH- steht und R^{x} für C₁-C₄ Alkyl steht.

Derartige Astaxanthin-Derivate und Verfahren zu ihrer Herstellung sind aus der WO 03/066583 A1 bekannt.

Dementsprechend betrifft die vorliegende Erfindung ein Verfahren zur Herstellung einer Lösung eines Astaxanthin-Derivats in einem für Nahrungsmittel geeigneten Öl, umfassend die folgenden Schritte:
i) Bereitstellen einer Suspension oder Lösung des Astaxanthin-Derivats in einem organischen, aus Sauerstoff, Wasserstoff und Kohlenstoff bestehenden, für Nahrungsmittel geeigneten Lösungsmittel, das unter C₂-C₆-Alkanolen, C₁-C₄-Alkylestern aliphatischer C₁-C₄-Carbonsäuren und aliphatischen Ketonen mit 3 bis 6 C-Atomen ausgewählt ist,
ii) Einarbeiten der Lösung oder Suspension des Astaxanthin-Derivats in ein für Nahrungsmittel geeignetes Öl, wobei man eine Lösung des Astaxanthin-Derivats erhält;
   wobei das Astaxanthin-Derivat zu wenigstens 70 Gew.-%, bevorzugt zu wenigstens 80 Gew.-%, insbesondere zu wenigstens 90 Gew.-% und speziell zu wenigstens 96 Gew.-% aus wenigstens einer Verbindung der Formel I besteht.

Die Erfindung ist mit einer Reihe von Vorteilen verbunden. Zum einen lassen sich unter Anwendung des erfindungsgemäßen Verfahrens in einfacher Weise Lösungen von Astaxanthin-Derivaten in flüssigen, für Nahrungsmittel geeigneten Ölen durch Verdünnen der in Schritt i) hergestellten Suspension oder Lösung des Astaxanthin-Derivats mit dem für Nahrungsmittel geeigneten Öl herstellen. Anders als in dem in WO 2006/125591 beschriebenen Verfahren bedarf es hierzu grundsätzlich nicht der Anwendung von Temperaturen oberhalb 100 °C. Vielmehr kann man mit dem erfindungsgemäßen Verfahren verdünnte Lösungen des Astaxanthin-Derivats in dem Öl bei Temperaturen von unterhalb 100 °C, insbesondere maximal 90 °C oder gar maximal 70 °C oder maximal 50 °C, z. B. bei Temperaturen im Bereich von 10 bis 90 °C insbesondere 20 bis 70 °C oder 30 bis 50 °C herstellen. Aufgrund der niedrigen Temperaturen zeichnen sich die auf diese Weise erhältlichen Lösungen des Astaxanthin-Derivats durch einen sehr hohen Anteil an all-trans-Isomeren aus, der in der Regel 70 %, häufig oberhalb 80 %, insbesondere oberhalb 90 %, bezogen auf das in der Formulierung enthaltene Astaxanthin-Derivat liegt. Ferner lassen sich unter Verwendung von Astaxanthin-Derivaten, die zu wenigstens 70 Gew.-% aus wenigstens einer Verbindungen der Formel I bestehen, sehr viel einfacher von Lösungen oder Suspensionen in den vorgenannten Lösungsmitteln herstellen als bei Verwendung von Astaxanthin selber oder von anderen, davon verschiedenen Astaxanthin-Derivaten wie Astaxanthindiacetat oder Astaxanthindisuccinat.

Die erfindungsgemäßen festen Formulierungen haben neben einer guten Verdünnbarkeit in den für Nahrungsmittel geeigneten Ölen den Vorteil einer verbesserten Handhabbarkeit und Stabilität im Vergleich zu den aus PCT/EP2008/061384 bekannten flüssigen Suspensionen.

In Formel I steht C₁-C₄-Alkyl für einen linearen oder verzweigten, gesättigten Kohlenwasserstoffrest mit 1 bis 4 C-Atomen wie Methyl, Ethyl, n-Propyl, 1-Methylethyl, Butyl, 1-Methylpropyl, 2-Methylpropyl oder 1,1-Dimethylethyl.

Die beiden Reste R in Formel I können gleich oder verschieden sein und sind vorzugsweise identisch.

Vorzugsweise steht A für 1,2-Ethandiyl. R^{x} steht vorzugsweise für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Die Verbindungen der Formel I können, da die die Gruppe O-C(O)R tragenden Kohlenstoffatome Asymmetriezentren sind, diastereomere und enantiomere Formen bilden, nämlich die 3R,3'S-Form, die 3S,3'R-Form, die 3R,3'R-Form und die 3S,3'S-Form. Sofern die Reste R in Formel I gleich sind, sind die 3R,3'S-Form und die 3S,3R-Form identisch und achiral (meso-Form) und die 3S,3'S-Form und die 3R,3'R-Form bilden ein Enantiomerenpaar. Für die Erfindung können sowohl die reinen Enantiomere und Diastereomere sowie Gemische der vorgenannten Diastereomere, z. B. ein racemisches Gemisch der 3S,3'S-Form und der 3R,3'R-Form als auch ein Gemisch der meso-Form mit der 3S,3'S-Form und/oder der 3R,3'R-Form oder ein Gemisch der meso-Form und dem Racemat der 3S,3'S-Form und der 3R,3'R-Form, eingesetzt werden. In einer bevorzugten Ausgestaltung der Erfindung wird die Verbindung der Formel I in der 3S,3'S-Form oder einer Mischung der 3S,3'S-Form mit einer oder weiteren Formen der Verbindung der Formel I eingesetzt, worin die 3S,3'S-Form wenigstens 80 Gew.-%, insbesondere wenigstens 90 Gew.-%, bezogen auf die Gesamtmenge der Verbindung der Formel I ausmacht. In einer weiteren bevorzugten Ausgestaltung der Erfindung wird die Verbindung der Formel I in der 3R,3'R-Form oder einer Mischung der 3R,3'R-Form mit einer oder weiteren Formen der Verbindung der Formel I eingesetzt, worin die 3R,3'R-Form wenigstens 80 Gew.-%, insbesondere wenigstens 90 Gew.-%, bezogen auf die Gesamtmenge der Verbindung der Formel I ausmacht.

Die in Schritt i) hergestellten Lösungen enthalten in der Regel 0,5 bis 50 Gew.-%, insbesondere 1 bis 30 Gew.-% und speziell 1,5 bis 25 Gew.-%, bezogen auf das Gesamtgewicht der Formulierung, wenigstens eines Astaxanthin-Derivats, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zubereitung, aus einer Astaxanthin-Verbindung der Formel I besteht. Im Falle der Suspensionen liegt die Konzentration vorzugsweise im Bereich von 1 bis 50 Gew.-%, insbesondere im Bereich von 2 bis 30 Gew.-% und speziell im Bereich von 3 bis 25 Gew.-%, bezogen auf das Gesamtgewicht der Suspension. Im Falle der Lösungen wird die Konzentration des Astaxanthin-Derivats in der Regel 10 Gew.-%, häufig 7 Gew.-% und insbesondere 5 Gew.-% nicht überschreiten und liegt typischerweise im Bereich von 0,5 bis 10 Gew.-%, häufig 1 bis 7 Gew.-% insbesondere bei 1,5 bis 5 Gew.-%.

In der Regel weist das in Schritt i) eingesetzte Astaxanthin-Derivat eine Reinheit, bezogen auf das in Formel I gezeigten all-trans-Isomer, von wenigstens 70 Gew.-%, häufig wenigstens 80 Gew.-%, vorzugsweise wenigstens 90 Gew.-% auf. Neben dem all-trans-Isomeren kann das Astaxanthin-Derivat auch Anteile an Astaxanthin-Verbindungen der Formel I enthalten, in denen eine oder mehrere Doppelbindungen der in Formel I dargestellten all-trans-Isomere cis-Konfiguration aufweisen. Die Gesamtmenge an all-trans-Isomer der Formel I und cis-Isomeren der Formel I beträgt in der Regel wenigstens 80 Gew.-%, häufig wenigstens 90 Gew.-%, insbesondere wenigstens 96 Gew.-%. Neben dem all-trans-Isomeren der Formel I und etwaigem cis-Isomeren kann das Astaxanthin-Derivat auch weitere Carotinoide umfassen, wobei der Anteil dieser Verunreinigungen in der Regel 30 Gew.-%, häufig 20 Gew.-%, insbesondere 10 Gew.-%, besonders bevorzugt 4 Gew.-%, bezogen auf die Gesamtmenge des eingesetzten Astaxanthin-Derivats nicht überschreitet. Bei diesen Verunreinigungen handelt es sich in erster Linie um Halbester des Astaxanthins, d. h. um Verbindungen der im Folgenden gezeigten Formel II 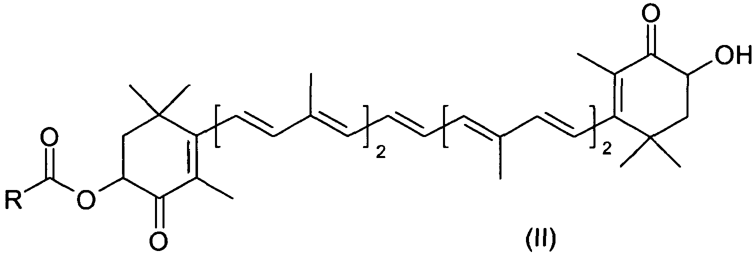 worin R die zuvor genannten Bedeutungen hat bzw. um cis-Isomere davon, sowie um Astaxanthin selber, Adonirubin und/oder Semi-Astacin.

Insbesondere wird in Schritt i) ein Astaxanthin-Derivat eingesetzt, das die an ein für Nahrungsmittel, insbesondere für Futtermittel zugelassenes Astaxanthin gestellten Anforderungen erfüllt, d. h. das weniger als 4 Gew.-% Carotinoide, die von Astaxanthin und seinen Derivaten verschieden sind, enthält und das einen Schwermetallgehalt von höchstens 10 ppm aufweist, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat, aus einer Astaxanthin-Verbindung der Formel I besteht, wobei das in Schritt i) eingesetzte Astaxanthin-Derivat zu wenigstens 70 Gew.-%, häufig wenigstens 80 Gew.-% und vorzugsweise wenigstens 90 Gew.-% aus dem all-trans-Isomeren besteht.

Bei den in Schritt a) hergestellten Zusammensetzungen handelt es sich um Suspensionen oder Lösungen des Astaxanthin-Derivats in einem für Nahrungsmittel geeigneten organischen Lösungsmittel.

Unter einer für Nahrungsmittel zugelassenen Substanz versteht man im Sinne der Erfindung eine für die Tier- und/oder Humanernährung zugelassene Substanz. Der Begriff "Nahrungsmittel" umfasst hier und im Folgenden Nahrungsmittel für die Humanernährung (= Lebensmittel) und Nahrungsmittel für die Tierernährung (= Futtermittel).

Erfindungsgemäß sind die organischen Lösungsmittel ausgewählt unter
- C₂-C₆-Alkanolen, wie beispielsweise Ethanol, 1-Propanol, 2-Propanol, 1-Butanol, 2-Butanol, 2-Methyl-1-propanol, 3-Methyl-1-butanol, 1-Pentanol und deren Gemische;
- C₁-C₄-Alkylestern aliphatischer C₁-C₄-Carbonsäuren, wie beispielsweise die Methyl-, Ethyl-, n-Propyl-, iso-Propyl-, n-Butyl-, 2-Butyl- oder iso-Butylester der Ameisensäure, der Essigsäure, der Propionsäure oder der Buttersäure, wie Methylacetat, Ethylacetat, n-Propylacetat, Isopropylacetat, n-Butylacetat, Isobutylacetat, Ethylformat und deren Gemische; sowie
- aliphatische, insbesondere nicht-cyclische Ketone mit 3 bis 6 C-Atomen wie Aceton, Methylethylketon, Isobutylmethylketon und deren Gemische; sowie
- Gemische der vorgenannten Lösungsmittel aus verschiedenen Klassen der vorgenannten Lösungsmittel.

Gemäß einer ersten Ausführungsform umfasst das in Schritt i) eingesetzte organische Lösungsmittel als Hauptbestandteil, das heißt zu wenigstens 50 Vol.-%, insbesondere zu wenigstens 70 Vol.-% und speziell zu wenigstens 90 Vol.-%, wenigstens eines der vorgenannten C₂-C₆-Alkanole.

Gemäß einer zweiten Ausführungsform umfasst das in Schritt i) eingesetzte organische Lösungsmittel als Hauptbestandteil, das heißt zu wenigstens 50 Vol.-%, insbesondere zu wenigstens 70 Vol.-% und speziell zu wenigstens 90 Vol.-%, wenigstens einen der vorgenannten C₁-C₄-Alkylester aliphatischer C₁-C₄-Carbonsäuren.

Gemäß einer dritten Ausführungsform umfasst das in Schritt i) eingesetzte organische Lösungsmittel als Hauptbestandteil, das heißt zu wenigstens 50 Vol.-%, insbesondere zu wenigstens 70 Vol.-% und speziell zu wenigstens 90 Vol.-%, wenigstens eines der vorgenannten Ketone.

Vorzugsweise weist das in Schritt i) eingesetzte organische Lösungsmittel weniger als 5 Vol.-%, bezogen auf die Gesamtmenge an Lösungsmittel, und insbesondere kein organisches Lösungsmittel auf, das von den vorgenannten organischen Lösungsmitteln verschieden ist. Das in Schritt i) eingesetzte organische Lösungsmittel kann geringe Mengen an Wasser enthalten, die vorzugsweise jedoch 10 Vol.-%, bezogen auf die Gesamtmenge an Lösungsmittel, nicht überschreiten.

In den in Schritt i) hergestellten Zusammensetzungen kann das Astaxanthin-Derivat in molekular-dispers gelöster Form und/oder in suspendierter Form vorliegen. Sofern das Astaxanthin-Derivat zumindest teilweise, in der Regel zu wenigstens 10 Gew.-%, häufig zu wenigstens 50 Gew.-%, insbesondere zu wenigstens 80 Gew.-% oder wenigstens 90 Gew.-%, bezogen auf die Gesamtmenge an Astaxanthin-Derivat, in suspendierter Form vorliegt, spricht man hier und im Folgenden von Suspensionen. In Abhängigkeit von der Löslichkeit des Astaxanthin-Derivats in dem verwendeten Lösungsmittel und der Temperatur der Suspension enthält die Suspension auch gelöste Anteile des Astaxanthin-Derivats, wobei die Konzentration an gelöstem Astaxanthin-Derivat dann in der Regel unterhalb 1000 ppm liegt.

Im Falle von Suspensionen des Astaxanthin-Derivats hat es sich als vorteilhaft erwiesen, wenn wenigstens 80 Gew.-%, insbesondere wenigstens 90 Gew.-% der Partikel des Astaxanthin-Derivats einen Partikeldurchmesser unterhalb 100 µm, insbesondere unterhalb 70 µm und speziell unterhalb 50 µm aufweisen. Hierbei hat es sich als vorteilhaft erwiesen, wenn die Partikel des Astaxanthin-Derivats in der Suspension einen volumenmittleren Teilchendurchmesser (D_{4,3}-Wert) im Bereich von 0,2 bis 50 µm, insbesondere im Bereich von 0,3 bis 30 µm und speziell im Bereich von 0,5 bis 20 µm aufweisen. Unter dem volumenmittleren Teilchendurchmesser versteht man den mittels Fraunhofer-Beugung an einer verdünnten 0,01 bis 0,1 gew.-%igen, speziell 0,05 gew.-%igen Suspension bestimmten volumenmittleren Teilchendurchmesser.

Neben dem Astaxanthin-Derivat können die in Schritt i) bereitgestellten Suspensionen oder Lösungen auch weitere Bestandteile enthalten, wie sie für diese Zwecke üblicherweise geeignet sind und angewendet werden. Hierzu zählen lipophile Dispergiermittel, Antioxidantien (Oxidationsstabilisatoren) und dergleichen. Beispiele für Antioxidantien sind Tocopherole wie α-Tocopherol, α-Tocopherolpalmitat, α-Tocopherolacetat, tert.-Butylhydroxytoluol, tert.-Butylhydroxyanisol, Ascorbinsäure, deren Salze und Ester wie z. B. Natriumascorbat, Calciumascorbat, Ascorbylphosphatester und Ascorbylpalmitat und Ethoxyquin. Die Antioxidantien sind, sofern erwünscht, in der erfindungsgemäßen Formulierung typischerweise in Mengen von 0,01 bis 10 Gew.-%, bezogen auf das Gesamtgewicht der in Schritt i) bereitgestellten Lösung oder Suspension, enthalten. Typische lipophile Dispergiermittel sind Ascorbylpalmitat, Polyglycerin-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester wie PEG(20)-Sorbitanmonooleat, Monoester der Milchsäure mit gesättigten C₁₀-C₂₄-Fettsäuren, Zuckerester von gesättigten C₁₆-C₂₈-Fettsäuren, Propylenglycol-Fettsäureester sowie Phospholipide wie Lecithin. Lipophile Dispergiermittel sind, sofern erwünscht, in der Zusammensetzung typischerweise in Mengen von 0,01 bis 10 Gew.-%, bezogen auf das Gesamtgewicht der in Schritt i) bereitgestellten Lösung oder Suspension, enthalten. Lipophile Dispergiermittel werden, sofern erwünscht, üblicherweise in Mengen von 0,1 bis 10 Gew.-Teilen, bezogen auf 1 Gew.-Teil des Astaxanthin-Derivats eingesetzt. In einer bevorzugten Ausführungsform der Erfindung enthält die in Schritt i) bereitgestellte Lösung oder Suspension im Wesentlichen kein, d. h. weniger als 0,5 Gew.-%, insbesondere weniger als 0,1 Gew.-%, bezogen auf das Gesamtgewicht der Lösung oder Suspension, lipophiles Dispergiermittel.

Die Herstellung der Lösung oder Suspension in dem organischen Lösungsmittel kann in an sich üblicher Weise durch Auflösen oder Suspendieren des Astaxanthin-Derivats und gegebenenfalls weiterer Bestandteile in dem organischen Lösungsmittel, gegebenenfalls unter Einwirken von Scherkräften und/oder Anwendung von erhöhten Temperaturen erfolgen.

Das zur Herstellung der Suspension bzw. Lösung eingesetzte organische Lösungsmittel kann Antioxidantien, z. B. Tocopherole wie α-Tocopherol oder Ascorbylpalmitat und/oder lipophile Dispergiermittel, vorzugsweise in den oben angegebenen Mengen, enthalten. Daneben kann das Lösungsmittel auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des eingesetzten Lösungsmittels enthalten.

Gemäß einer bevorzugten Ausführungsform erfolgt die Bereitstellung der Suspension durch Vermahlen des Astaxanthin-Derivats, welches die oben genannte Reinheit aufweist, in dem organischen Lösungsmittel. Hierzu wird man in der Regel das Astaxanthin-Derivat zunächst in dem organischen Lösungsmittel suspendieren, wobei man eine grobteilige Suspension des Astaxanthin-Derivats erhält und anschließend die Partikel in einer geeigneten Vorrichtung zum Vermahlen auf die gewünschte Teilchengrö-βe zerkleinern. Als Vorrichtungen zum Vermahlen können übliche, dem Fachmann bekannte Vorrichtungen, beispielsweise Kugelmühlen, Perlmühlen oder KolloidMühlen, eingesetzt werden, die gegebenenfalls kühlbar sind.

Das Vermahlen erfolgt typischerweise bei Temperaturen unterhalb des Siedepunkts der organischen Lösungsmittel, insbesondere unterhalb 70 °C oder unterhalb 50 °C, häufig im Bereich von 10 bis 70 °C und insbesondere im Bereich von 20 bis 50 °C. Gegebenenfalls wird man das Vermahlen unter Kühlung durchführen.

Das Vermahlen wird so lange durchgeführt, bis die gewünschte Teilchengröße erreicht ist. Vorzugsweise wird die Vermahlung so lange fortgesetzt, bis die Partikel des Astaxanthin-Derivats in der Suspension einen volumenmittleren Teilchendurchmesser im Bereich von 0,2 bis 50 µm, insbesondere im Bereich von 0,3 bis 30 µm und speziell im Bereich von 0,5 bis 20 µm aufweisen.

Die in Schritt i) hergestellte Lösung oder Suspension des Astaxanthin-Derivats wird in Schritt ii) in ein für Nahrungsmittel geeignetes, vorzugsweise unter Prozessbedingungen flüssiges Öl eingearbeitet, wobei man eine Lösung des Astaxanthin-Derivats erhält. Hierzu wird man die Lösung oder Suspension aus Schritt i) in einer Menge einsetzen, dass ein Gehalt an Astaxanthin-Derivat in der Lösung des Astaxanthin-Derivats in dem für Nahrungsmittel geeigneten Öl im Bereich von 10 bis 5000 ppm, insbesondere 20 bis 2000 ppm vorzugsweise 50 bis 1000 ppm resultiert.

Unter "Prozessbedingungen flüssig" bedeutet, dass das für Nahrungsmittel geeignete Öl unter den Bedingungen des Einarbeitens der in Schritt ii) erhaltenen Lösung in die Bestandteile der Futtermittelzusammensetzung flüssig ist.

Das in Schritt ii) eingesetzte Öl kann synthetischer, mineralischer, pflanzlicher oder tierischer Herkunft sein. Beispiel sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl.

Bevorzugt sind pflanzliche Öle und Öle tierischen Ursprungs, die bei 40 °C flüssig sind, insbesondere Pflanzenöle wie Sojabohnenöl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

In einer bevorzugten Ausführungsform der Erfindung umfasst das in Schritt ii) eingesetzte Öl zu wenigstens 50 Gew.-% und insbesondere zu wenigstens 80 Gew.-%, bezogen auf die Gesamtmenge des in Schritt ii) eingesetzten essbaren Öls, wenigstens ein unter Maisöl, Sonnenblumenöl, Sojabohnenöl und Fischöl ausgewähltes essbares Öl.

Bei den in Schritt ii) eingesetzten Ölen kann es sich um Raffinatöle oder Rohöle handeln, die noch ursprungsspezifische Verunreinigungen wie Proteine, Phosphat, (Erd-)Alkalimetallsalze und dergleichen in üblichen Mengen enthalten. Die in Schritt ii) eingesetzten Öle können auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des in Schritt ii) eingesetzten Öls, enthalten.

Das Einarbeiten der Suspension oder Lösung aus Schritt i) in das für Nahrungsmittel geeignete Öl erfolgt in der Regel durch Vermischen bei Temperaturen unterhalb 100 °C, insbesondere maximal 80 °C oder maximal 70 °C oder maximal 50 °C. Das Einarbeiten der in Schritt i) erhaltenen Zusammensetzung in das flüssige Öl erfolgt typischerweise bei Temperaturen im Bereich von 15 bis 80 °C, insbesondere im Bereich von 20 bis 70 °C und speziell im Bereich von 30 bis 50 °C.

Gegebenenfalls wird man das Öl, in welches man die in Schritt i) erhaltene Zusammensetzung des Astaxanthin-Derivats einarbeitet, erwärmen. In der Regel liegt die Temperatur des erwärmten Öls vorzugsweise maximal bei 100 °C und beträgt insbesondere nicht mehr als 80 °C oder 70 °C und speziell nicht mehr als 50 °C, und liegt vorzugsweise im Bereich von 25 bis 80 °C, insbesondere im Bereich von 30 bis 70 °C und speziell im Bereich von 30 bis 50 °C.

Beispielsweise kann man so vorgehen, dass man ein für Nahrungsmittel geeignetes Öl, das vorzugsweise einen Schmelzpunkt von nicht mehr als 40 °C aufweist, in einem geeigneten Gefäß vorlegt, gegebenenfalls auf die gewünschte Temperatur vorwärmt und hierzu die in Schritt i) hergestellte Zusammensetzung unter Durchmischen zugibt, wobei die Zugabe in einer Portion, in mehreren Portionen oder kontinuierlich erfolgen kann. Die zugegebene Zusammensetzung des Astaxanthin-Derivats kann auf die gewünschte Temperatur vorgewärmt werden, was jedoch grundsätzlich nicht erforderlich ist.

Das Einarbeiten der in Schritt i) hergestellten Zusammensetzung in das für Nahrungsmittel geeignete Öl kann auch kontinuierlich durch so genannte Inline-Mischer, d. h. kontinuierlich arbeitende Mischvorrichtungen, z. B. statische Mischer oder dynamische Mischer, erfolgen. Beispiele für geeignete statische Mischer sind Mischkammern mit oder ohne Einbauten, Mischrohre mit oder ohne Mischdüsen, Jet-Mischer und der gleichen. Beispiele für dynamische Mischer sind Rotor-Stator-Mischer, Mischkammern mit Rührwerken, Mischpumpen und dergleichen. Gegebenenfalls schließt sich der Mischvorrichtung noch eine Verweilzone an, die gegebenenfalls temperiert werden kann, um das Lösen des Astaxanthin-Derivats zu vervollständigen.

Die zum Erreichen des Lösens erforderliche Mischdauer hängt naturgemäß von der Temperatur, dem gewählten Öl und den apparativen Gegebenheiten ab und liegt typischerweise im Bereich von 10 sec bis 120 min. Der Fachmann kann die erforderliche Mischzeit durch Routineexperimente ermitteln.

Im Anschluss an das Mischen kann man die erhaltene ölige Lösung, sofern erforderlich, abkühlen, z. B. mittels geeigneter Wärmetauscher oder durch Verdünnen mit einem kalten essbaren Öl, das zu dem zum Lösen in Schritt ii) eingesetzten Öl gleich oder verschieden sein kann.

Gegebenenfalls kann man während oder im Anschluss an das Einarbeiten der Suspension bzw. Lösung einen Teil oder die Gesamtmenge des organischen Lösungsmittels entfernen, z. B. durch Anlegen von Vakuum. Das so entfernte Lösungsmittel kann rückgeführt werden, z. B. zur Herstellung der Suspension oder Lösung in Schritt i).

Auf diese Weise erhält man stabile Lösungen von Astaxanthin-Derivaten in für Nahrungsmittel geeigneten Ölen. Diese Lösungen zeichnen sich dadurch aus, dass das in ihnen enthaltene Astaxanthin-Derivat einen hohen Anteil an all-trans-Isomeren aufweist, der in der Regel oberhalb 80 %, insbesondere oberhalb 90 % liegt.

Die so erhaltenen Lösungen enthalten neben dem Astaxanthin-Derivat die in der Formulierung enthaltenen Hilfsstoffe, wie ggf. Oxidationsstabilisatoren, lipophile Dispergiermittel, Fließhilfsmittel und/oder Verdicker.

Es versteht sich von selber, dass die in Schritt ii) erhaltenen Lösungen geringe Mengen an Wasser aus dem zu ihrer Herstellung eingesetzten, für Nahrungsmittel Öl enthalten können. Häufig jedoch beträgt der Wasseranteil nicht mehr als 10 Gew.-%, z. B. 0,1 bis 10 Gew.-%, häufig 0,5 bis 8 Gew.-%, und in einer speziellen Ausführungsform der Erfindung nicht mehr als 0,5 Gew.-%, bezogen auf das in der Lösung enthaltene Öl.

Die nach dem erfindungsgemäßen Verfahren erhältlichen Lösungen des Astaxanthin-Derivats sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau, kommt. Insbesondere zeichnen sie sich durch einen hohen Anteil an all-trans-Isomeren des eingesetzten Astaxanthin-Derivats aus und durch einen vergleichsweise geringen Anteil an Abbauprodukten des Astaxanthin-Derivats.

Das erfindungsgemäße Verfahren wird häufig direkt in die Prozesse zur Weiterverarbeitung der Lösungen des Astaxanthin-Derivats integriert werden.

Die nach dem erfindungsgemäßen Verfahren erhältlichen Lösungen des Astaxanthin-Derivats eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Insbesondere eignen sich die erfindungsgemäß erhältlichen öligen Lösungen zur Herstellung von Futtermittelzubereitungen, insbesondere von festen Futtermittelzubereitungen, speziell von Pellets.

Bevorzugt lassen sich die Lösungen als Futtermittelzusatz in der Tieremährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als so genannte "post-pelleting liquid application". Vorzugsweise belädt man die Futtermittelpellets mit den Formulierungen unter vermindertem Druck.

Dementsprechend betrifft die vorliegende Erfindung auch die Herstellung von Futtermittel, wie Tierfuttermittel, Nahrungsmittel für die Humanernährung, Nahrungsergänzungsmittel, sowie die Herstellung pharmazeutischer Mittel oder kosmetischer Mittel unter Verwendung der erfindungsgemäß hergestellten Lösungen des Astaxanthin-Derivats. Diese Mittel enthalten neben den für diese Mittel üblichen Bestandteilen das Öl, gegebenenfalls das organische Lösungsmittel und das Astaxanthin-Derivat. Dementsprechend betrifft die Erfindung auch ein Verfahren zur Herstellung von Futtermittelzubereitungen, insbesondere von festen Futtermittelzubereitungen, speziell von Pellets, umfassend:
a) Bereitstellung einer Lösung eines Astaxanthin-Derivats in einem für Nahrungsmittel geeigneten Öl nach einem Verfahren gemäß einem der vorhergehenden Ansprüche;
c) Vermischen der in Schritt a) erhaltenen flüssigen Lösung mit den Bestandteilen der Futtermittelzubereitung oder Imprägnieren einer festen Futtermittelzubereitung mit der in Schritt a) erhaltenen Lösung.

Bevorzugte Ausführungsformen betreffen Tierfuttermittel, insbesondere Fischfuttermittel, die das flüssige Öl, das feste Verdünnungsmittel und das Astaxanthin-Derivat umfassen. Derartige Mittel enthalten das in der Lösung enthaltene Astaxanthin-Derivat typischerweise in einer Menge von 10 bis 100 ppm, bezogen auf das Gesamtgewicht des Mittels, wobei das Astaxarithin-Derivat in der Regel zu mehr als 70 %, insbesondere zu wenigstens 80 % und speziell zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Astaxanthin-Derivats beladen sind. Derartige Pellets enthalten das in der Lösung enthaltene Astaxanthin-Derivat typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäß erhältlichen Lösung des Astaxanthin-Derivats, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut die Lösung des Astaxanthin-Derivats oder ein für Nahrungsmittel geeignetes, flüssiges Öl in der zuvor beschriebenen Weise aufsprühen. Auf diese Weise erhält man Pellets, die im Kern das Öl und das Astaxanthin-Derivat enthalten.

## Patentansprüche

1. Verfahren zur Herstellung einer Lösung eines Astaxanthin-Derivats in einem für Nahrungsmittel geeigneten Öl, umfassend
i) Bereitstellen einer Suspension oder Lösung des Astaxanthin-Derivats in einem organischen, aus Sauerstoff, Wasserstoff und Kohlenstoff bestehenden, für Nahrungsmittel geeigneten Lösungsmittel, das unter C₂-C₆-Alkanolen, C₁-C₄-Alkylestern aliphatischer C₁-C₄-Carbonsäuren und aliphatischen Ketonen mit 3 bis 6 C-Atomen ausgewählt ist,
ii) Einarbeiten der Lösung oder Suspension des Astaxanthin-Derivats in ein für Nahrungsmittel geeignetes Öl, wobei man eine Lösung des Astaxanthin-Derivats erhält;
wobei das Astaxanthin-Derivat zu wenigstens 70 Gew.-% aus wenigstens einer Verbindung der Formel I 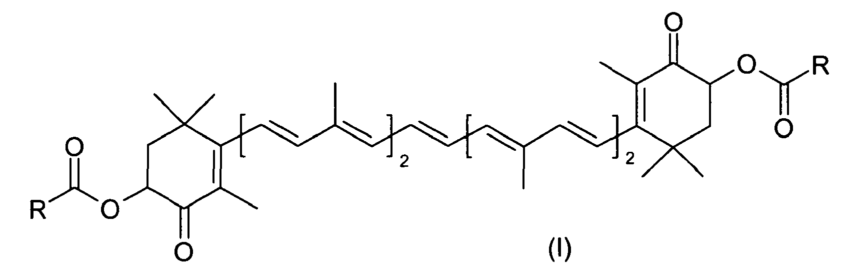 besteht, worin R für einen Rest der Formel 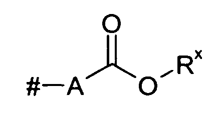 steht, worin # die Anknüpfung an die Carbonylgruppe bedeutet, A für -CH₂CH₂- oder für -CH=CH- steht und R^{x} für C₁-C₄ Alkyl steht.

2. Verfahren nach Anspruch 1, wobei in Formel I der Rest R^{x} Methyl oder Ethyl bedeutet und A für -CH₂CH₂- steht.

3. Verfahren nach einem der vorhergehenden Ansprüche, wobei das Astaxanthin-Derivat in dem organischen Lösungsmittel in molekular-dispers gelöster Form oder in Form einer Suspension vorliegt, worin wenigstens 90 Gew.-% der Partikel des Astaxanthin-Derivats einen Partikeldurchmesser unterhalb 100 µm aufweisen.

4. Verfahren nach einem der vorhergehenden Ansprüche, wobei das Astaxanthin-Derivat in dem organischen Lösungsmittel in Form einer Suspension vorliegt, worin der volumenmittlere Teilchendurchmesser der Partikel des Astaxanthin-Derivats im Bereich von 0,1 bis 50 µm liegt.

5. Verfahren nach einem der vorhergehenden Ansprüche, wobei das organische Lösungsmittel unter C₁-C₆-Alkanolen ausgewählt ist.

6. Verfahren nach Anspruch 5, wobei das organische Lösungsmittel unter Ethanol, 1-Propanol, 2-Propanol, 1-Butanol, 2-Butanol, 2-Methyl-1-propanol, 3-Methyl-1-butanol, 1-Pentanol und deren Gemischen ausgewählt ist.

7. Verfahren nach einem der Ansprüche 1 bis 4, wobei das organische Lösungsmittel unter C₁-C₄-Alkylestern aliphatischer C₁-C₄-Carbonsäuren ausgewählt ist.

8. Verfahren nach Anspruch 7, wobei das organische Lösungsmittel unter Methylacetat, Ethylacetat, n-Propylacetat, Isopropylacetat, n-Butylacetat, Isobutylacetat, Ethylformat und deren Gemischen ausgewählt ist.

9. Verfahren nach einem der Ansprüche 1 bis 4, wobei das organische Lösungsmittel unter Ketonen mit 3 bis 6 C-Atomen ausgewählt ist.

10. Verfahren nach Anspruch 9, wobei das organische Lösungsmittel unter Aceton, Methylethylketon, Isobutylmethylketon und deren Gemischen ausgewählt ist.

11. Verfahren nach einem der vorhergehenden Ansprüche, wobei die in Schritt i) hergestellte Lösung oder Suspension einem Gehalt an Astaxanthin-Derivat von 0,5 bis 50 Gew.-%, bezogen auf das Gesamtgewicht der Lösung bzw. Suspension aufweist.

12. Verfahren nach einem der vorhergehenden Ansprüche, wobei die Verbindung der Formel I in der Formulierung zu wenigstens 80 % in der all-trans-Form vorliegt.

13. Verfahren nach einem der vorhergehenden Ansprüche, wobei man in Schritt ii) die Lösung oder Suspension aus Schritt i) in einer Menge einsetzt, dass ein Gehalt an Astaxanthin-Derivat in der Lösung des Astaxanthin-Derivats in dem für Nahrungsmittel geeigneten Öl im Bereich von 10 bis 2000 ppm resultiert.

14. Verfahren nach einem der vorhergehenden Ansprüche, wobei das in Schritt ii) eingesetzte, für Nahrungsmittel geeignete Öl ausgewählt ist unter Sojabohnenöl, Palmöl, Palmkernöl, Sonnenblumenöl, PUFA-Ölen, MCT-Ölen, Fischölen, Distelöl, Maisöl, Olivenöl, Leinsamenöl, Rapsöl, Reisöl und Mischungen dieser Öle.

15. Verfahren zur Herstellung von Futtermittelzubereitungen, insbesondere von festen Futtermittelzubereitungen, speziell von Pellets, umfassend:
a) Bereitstellung einer Lösung eines Astaxanthin-Derivats in einem für Nahrungsmittel geeigneten Öl nach einem Verfahren gemäß einem der vorhergehenden Ansprüche;
b) Vermischen der in Schritt a) erhaltenen flüssigen Lösung mit den Bestandteilen der Futtermittelzubereitung oder Imprägnieren einer festen Futtermittelzubereitung mit der in Schritt a) erhaltenen Lösung.

## Claims

1. A method for producing a solution of an astaxanthin derivative in an oil suitable for foods, which comprises
i) providing a suspension or solution of the astaxanthin derivative in an organic solvent suitable for foods and comprising oxygen, hydrogen and carbon which is selected from C₂-C₆ alkanols, C₁-C₄ alkyl esters of aliphatic C₁-C₄ carboyxlic acids and aliphatic ketones having 3 to 6 carbon atoms,
ii) incorporating the solution or suspension of the astaxanthin derivative into an oil suitable for foods, wherein a solution of the astaxanthin derivative is obtained;
wherein the astaxanthin derivative comprises at least 70% by weight of at least one compound of the formula I 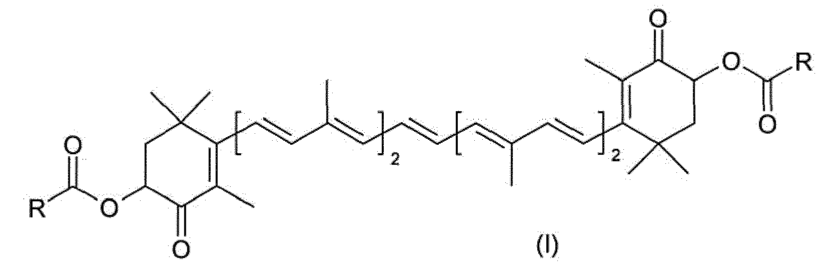 where R is a moiety of the formula 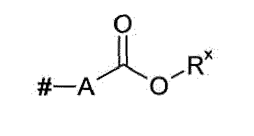 where # means the link to the carbonyl group, A is -CH₂CH₂- or -CH=CH-and R^{x} is C₁-C₄ alkyl.

2. The method according to claim 1, wherein in formula the moiety R^{x} is methyl or ethyl and A is -CH₂CH₂-.

3. The method according to any one of the preceding claims, wherein the astaxanthin derivative is present in the organic solvent in molecularly disperse dissolved form or in the form of a suspension, where at least 90% by weight of the particles of the astaxanthin derivative have a particle diameter below 100 µm.

4. The method according to any one of the preceding claims, wherein the astaxanthin derivative is present in the organic solvent in the form of a suspension, where the volume-mean particle diameter of the particles of the astaxanthin derivative is in the range from 0.1 to 50 µm.

5. The method according to any one of the preceding claims, wherein the organic solvent is selected from C₁-C₆ alkanols.

6. The method according to claim 5, wherein the organic solvent is selected from ethanol, 1-propanol, 2-propanol, 1-butanol, 2-butanol, 2-methyl-1-propanol, 3-methyl-1 -butanol, 1 -pentanol and mixtures thereof.

7. The method according to any one of claims 1 to 4, wherein the organic solvent is selected from C₁-C₄ alkyl esters of aliphatic C₁-C₄ carboxylic acids.

8. The method according to claim 7, wherein the organic solvent is selected from methyl acetate, ethyl acetate, n-propyl acetate, isopropyl acetate, n-butyl acetate, isobutyl acetate, ethyl formate and mixtures thereof.

9. The method according to any one of claims 1 to 4, wherein the organic solvent is selected from ketones having 3 to 6 carbon atoms.

10. The method according to claim 9, wherein the organic solvent is selected from acetone, methyl ethyl ketone, isobutyl methyl ketone and mixtures thereof.

11. The method according to any one of the preceding claims, wherein the
solution or suspension produced in step i) has a content of astaxanthin derivative of 0.5 to 50% by weight, based on the total weight of the solution or suspension.

12. The method according to any one of the preceding claims, wherein the compound of the formula I is present at at least 80% in the all-trans form in the formulation.

13. The method according to any one of the preceding claims, wherein, in step ii), the solution or suspension of step i) is used in an amount such that a content of astaxanthin derivative in the solution of the astaxanthin derivative in the oil suitable for foods in the range from 10 to 2000 ppm results,

14. The method according to any one of the preceding claims, wherein the oil suitable for foods used in step ii) is selected from soybean oil, palm oil, palm kernel oil, sunflower oil, PUFA oils, MCT oils, fish oils, safflower oil, corn oil, olive oil, linseed oil, rapeseed oil, rice oil and mixtures of these oils.

15. A method for producing feed preparations, in particular solid feed preparations, especially pellets, which comprises:
a) providing a solution of an astaxanthin derivative in an oil suitable for foods by a method according to any one of the preceding claims;
b) mixing the liquid solution obtained in step a) with the components of the feed preparation or impregnating a solid feed preparation with the solution obtained in step a).

## Revendications

1. Procédé de fabrication d'une solution d'un dérivé d'astaxanthine dans une huile appropriée pour les denrées alimentaires, comprenant :
i) la préparation d'une suspension ou d'une solution du dérivé d'astaxanthine dans un solvant organique, constitué d'oxygène, d'hydrogène et de carbone, approprié pour les denrées alimentaires, choisi parmi les alcanols en C₂-C₆, les esters alkyliques en C₁-C₄ d'acides carboxyliques aliphatiques en C₁-C₄ et les cétones aliphatiques contenant 3 à 6 atomes C,
ii) l'incorporation de la solution ou suspension du dérivé d'astaxanthine dans une huile appropriée pour les denrées alimentaires, une solution du dérivé d'astaxanthine étant obtenue ;
le dérivé d'astaxanthine étant constitué à au moins 70 % en poids d'au moins un composé de formule I 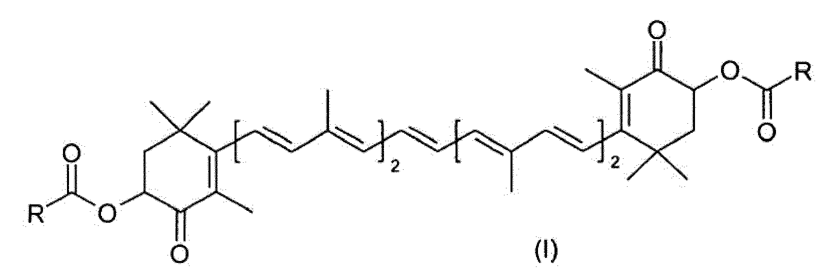 dans lequel R représente un radical de formule 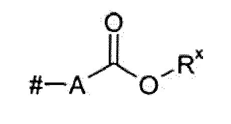 dans laquelle # signifie la liaison au groupe carbonyle, A représente -CH₂CH₂- ou -CH=CH- et R^{x} représente alkyle en C₁-C₄.

2. Procédé selon la revendication 1, dans lequel, dans la formule I, le radical R^{x} signifie méthyle ou éthyle et A représente -CH₂CH₂-.

3. Procédé selon l'une quelconque des revendications précédentes, dans lequel le dérivé d'astaxanthine se présente dans le solvant organique sous forme dissoute dispersée moléculairement ou sous la forme d'une suspension dans laquelle au moins 90 % en poids des particules du dérivé d'astaxanthine présentent un diamètre de particule inférieur à 100 µm.

4. Procédé selon l'une quelconque des revendications précédentes, dans lequel le dérivé d'astaxanthine se présente dans le solvant organique sous la forme d'une suspension dans laquelle le diamètre de particule moyen en volume des particules du dérivé d'astaxanthine se trouve dans la plage allant de 0,1 à 50 µm.

5. Procédé selon l'une quelconque des revendications précédentes, dans lequel le solvant organique est choisi parmi les alcanols en C₁-C₆.

6. Procédé selon la revendication 5, dans lequel le solvant organique est choisi parmi l'éthanol, le 1-propanol, le 2-propanol, le 1-butanol, le 2-butanol, le 2-méthyl-1-propanol, le 3-méthyl-1-butanol, le 1-pentanol et leurs mélanges.

7. Procédé selon l'une quelconque des revendications 1 à 4, dans lequel le solvant organique est choisi parmi les esters alkyliques en C₁-C₄ d'acides carboxyliques aliphatiques en C₁-C₄.

8. Procédé selon la revendication 7, dans lequel le solvant organique est choisi parmi l'acétate de méthyle, l'acétate d'éthyle, l'acétate de n-propyle, l'acétate d'isopropyle, l'acétate de n-butyle, l'acétate d'isobutyle, le formate d'éthyle et leurs mélanges.

9. Procédé selon l'une quelconque des revendications 1 à 4, dans lequel le solvant organique est choisi parmi les cétones contenant 3 à 6 atomes C.

10. Procédé selon la revendication 9, dans lequel le solvant organique est choisi parmi l'acétone, la méthyléthylcétone, l'isobutylméthylcétone et leurs mélanges.

11. Procédé selon l'une quelconque des revendications précédentes, dans lequel la solution ou suspension fabriquée à l'étape i) présente une teneur en dérivé d'astaxanthine de 0,5 à 50 % en poids, par rapport au poids total de la solution ou suspension.

12. Procédé selon l'une quelconque des revendications précédentes, dans lequel le composé de formule I se présente à au moins 80 % sous la forme all-trans dans la formulation.

13. Procédé selon l'une quelconque des revendications précédentes, dans lequel, à l'étape ii), la solution ou suspension de l'étape i) est utilisée en une quantité que résulte en une teneur en dérivé d'astaxanthine dans la solution du dérivé d'astaxanthine dans l'huile appropriée pour les denrées alimentaires dans la plage allant de 10 à 2 000 ppm.

14. Procédé selon l'une quelconque des revendications précédentes, dans lequel l'huile appropriée pour les denrées alimentaires utilisée à l'étape ii) est choisie parmi l'huile de soja, l'huile de palme, l'huile de palmiste, l'huile de tournesol, les huiles PUFA, les huiles MCT, les huiles de poissons, l'huile de carthame, l'huile de maïs, l'huile d'olive, l'huile de lin, l'huile de colza, l'huile de riz et les mélanges de ces huiles.

15. Procédé de fabrication de préparations d'aliments pour animaux, notamment de préparations d'aliments pour animaux solides, notamment de pastilles, comprenant :
a) la préparation d'une solution d'un dérive d'astaxanthine dans une huile appropriée pour les denrées alimentaires selon un procédé selon l'une quelconque des revendications précédentes ;
b) le mélange de la solution liquide obtenus à l'étape a) avec les constituants de la préparation d'aliment pour animaux ou l'imprégnation d'une préparation d'aliment pour animaux solide avec la solution obtenue à l'étape a).
